# EUROPEAN PATENT APPLICATION

(11) **EP 4 613 751 A1**
(43) Date of publication of application: **10.09.2025**
(21) Application number: 24162126.7
(22) Date of filing: 07.03.2024
(51) Int. Cl.: C07D 489/12

(54) **METHOD OF MAKING CHEMICAL COMPOUNDS USEFUL FOR THE SYNTHESIS OF BUPRENORPHINE**

(71) Applicant: Siegfried AG, 4800 Zofingen (CH)
(72) Inventor: Debnar, Thomas, 6010 Kriens (CH); Petzold, Daniel, 6260 Hintermoos (Wikon) (CH)
(74) Representative: Eisenführ Speiser

(57) **Abstract**

Described herein is a method of making chemical compounds useful in pharmaceutical syntheses, e.g. chemical compounds useful as intermediates in or final products of such syntheses, and in particular a method of making chemical compounds useful for the synthesis of buprenorphine, including buprenorphine. In one preferred variant, it is described herein a method of making buprenorphine from oripavine.

## Description

The present invention relates to a method of making chemical compounds useful in pharmaceutical syntheses, e.g. chemical compounds useful as intermediates in or final products of such syntheses, and in particular to a method of making chemical compounds useful for the synthesis of buprenorphine, including buprenorphine. In one preferred variant, the present invention relates to a method of making buprenorphine from oripavine.

Buprenorphine, also known as (5R,6R,7 R,9R, 13S, 14S)-17-cyclopropylmethyl-7-[(S)-3,3-dimethyl-2-hydroxybutan-2-yl]-6-methoxy-4,5-epoxy-6,14-ethanomorphinan-3-ol (IUPAC) is a potent semi-synthetic opioid analgesic, useful for the relief of moderate, chronic and acute pain, as well as in the therapy of opioid addiction, and is usually administered in the form of its hydrochloride salt. Since its approval, it has been marketed as injectable solution, various types of tablets, as well as in the form of patches. Buprenorphine can be administered as sole active ingredient, or in combination with other substances such as e.g. naloxone.

Document EP 1 368 023 A1 deals with the use of buprenorphine and its stereoisomers in the treatment of urinary incontinence.

In document WO 2013/042054 A1, the use of buprenorphine and its stereoisomers in the treatment of acute suicidality is described.

Recent studies by Greedy et al. (cf. Greedy B. M., et al., J. Med. Chem. 56 (2013) 3207-3216) showed the differentiated potency of stereoisomers of buprenorphine and analogues thereof, for example as orvinols having mixed κ/µ [kappa/mu] opioid receptor agonist activity. In one aspect, the capability to treat cocaine abuse has been shown. In this article, a process of manufacturing certain target molecules is also disclosed, where, however, said process requires several steps for positioning relevant substituents so that a desired stereochemical configuration is obtained.

Several further methods are known for synthesizing buprenorphine, its analogues and its stereoisomers, from compounds isolated from the opium poppy or compounds derived therefrom. Several of these methods use thebaine or oripavine as starting materials.

EP 1 439 179 A1 (corresponding to WO 03/024972 A1) discloses a classical route of synthesis from thebaine to buprenorphine and to analogues thereof. The synthetic route is a series of chemical reaction steps, including addition of a tert.-butyl group by a Grignard reaction.

WO 2007/081506 A1 describes the use of oripavine as a starting material for the synthesis of buprenorphine.

Further suggestions for opioid syntheses can be found in documents WO 2010/067101 A1 WO 2010/039221 A1, WO 2014/102591 A1, US 2014/0057932 A1 and CZ300995 B6.

Document WO 2008/048957 A1 and related document EP 2 332 410 A1 both discuss a process for preparing oxymorphone, naltrexone and buprenorphine, in each case using oripavine as starting material. A certain lack of more specific technical information in these documents, however, makes it difficult to transfer the methods disclosed therein to an industrial scale. Moreover, the use of halogenated organic solvents in the methods disclosed in these documents is increasingly incompatible with today's stricter environmental regulations.

In the light of the existing prior art, there is therefore a need for a simple, efficient and sustainable method of making chemical compounds useful in pharmaceutical syntheses, e.g. chemical compounds useful as intermediates in or final products of such syntheses, and in particular for a method of making chemical compounds useful for the synthesis of buprenorphine, including buprenorphine. Aspects of such simple, efficient and sustainable method concern the use of a reduced number of solvents and a reduced use or the avoidance of potentially harmful and/or hazardous solvents like halogenated organic solvents, while maintaining or increasing robustness of the method, in particular in terms of repeatability and reproducibility, as well as its suitability for larger-scale industrial use.

Correspondingly, it was a primary object of the present invention to provide a simple, efficient and sustainable method of making chemical compounds useful in pharmaceutical syntheses, e.g. chemical compounds useful as intermediates in, or final products of such syntheses, and in particular for a method of making chemical compounds useful for the synthesis of buprenorphine, including buprenorphine.

It has now been found that the primary object and other objects of the present invention can be accomplished by a method of making one or more chemical compounds useful in pharmaceutical syntheses, preferably useful in a method of making buprenorphine from oripavine, the method comprising the following step:
S2) providing or preparing a compound of formula VI 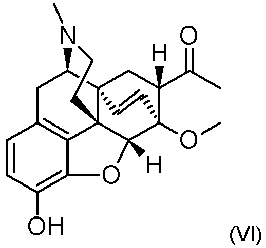
and reacting the compound of formula VI
in a liquid reaction medium comprising one or more non-halogenated (and preferably non-aromatic) organic solvents, in a total amount of ≥ 85 wt.-%, relative to the total weight of the liquid reaction medium,
   wherein the one or more non-halogenated (and preferably non-aromatic) organic solvents are selected from the group consisting of N,N-dimethyl formamide, γ-butyrolactone, N-methyl-2-pyrrolidone, N-butyl-2-pyrrolidone and dihydrolevoglucosenon,
in the presence of a base,
   preferably at a temperature not exceeding 40 °C, more preferably at a temperature not exceeding 30 °C,
   and
in the presence of a phase transfer catalyst,
   preferably in a total amount in the range of 1 to 10 mol-%, preferably of 1.5 to 7 mol-%, relative to the molar amount of compound of formula VI used in step S2),
with an agent suitable for introducing, to the free hydroxyl group of the compound of formula VI, a protective group PG which can be removed by hydrogenolysis,
to receive a compound of formula V 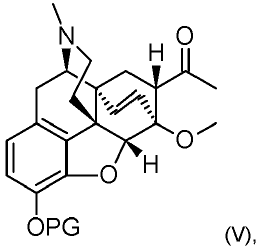
wherein PG is a protective group which can be removed by hydrogenolysis.

The invention as well as preferred variants and preferred combinations of parameters, properties and elements thereof are defined in the appended claims. Preferred aspects, details, modifications and advantages of the present invention are also defined and explained in the following description and in the examples shown below.

The present inventors have now found that the method according to the present invention, including its preferred variants as disclosed herein, is simple, efficient and sustainable and is excellently suited for producing intermediate compounds useful in pharmaceutical syntheses, or final products of such syntheses. In particular, the method according to the present invention, including its preferred variants, facilitates producing said intermediates or final products, in particular buprenorphine, and allows to reduce or to avoid the use of potentially harmful and/or hazardous solvents like halogenated organic solvents and/or to reduce the number of different solvents required, when compared with similar methods of the prior art. In addition, the method according to the present invention, including its preferred variants, shows a comparable or increased robustness, in particular in terms of repeatability and reproducibility, when compared with similar methods from the prior art, as well as an excellent suitability for larger-scale industrial application, e.g. for upscaling to a scale of about 500 kg of final product, e.g. of buprenorphine.

Reacting, in step S2) described above, the compound of formula VI with an agent suitable for introducing a protective group PG which can be removed by hydrogenolysis for protecting the free hydroxy group of the compound of formula VI does not need to be conducted in a liquid reaction medium comprising halogenated organic solvents or aromatic solvents like toluene. Instead, it was found that step S2) can be conducted in a liquid reaction medium comprising or consisting of one or more non-halogenated, polar-aprotic (and non-aromatic) organic solvents, as defined above.

As used herein, the term "liquid reaction medium" means the constituents of a reaction mixture of a certain reaction step (i.e. a reaction step of the different reaction steps S1) to S6) which are described herein in more detail) which are liquid at room temperature (25 °C) and normal pressure (1013 hPa) and which are inert under the respective reaction conditions. In particular, the term "liquid reaction medium" as described herein comprises the solvent or mixture of solvents which is used in a certain reaction step (i.e. a reaction step of the reaction steps S1) to S6), which are described herein in more detail). A liquid constituent of a reaction mixture in a certain of said reaction steps, which is involved (i.e. which takes part and functions as a reactant) in the reaction of said reaction step, is, however, not counted as part of the respective "liquid reaction medium" of said certain step.

Once any of compounds VI, V, IV, III, II or I has been formed, preferably once the formation of any of compounds VI, V, IV, III, II or I is completed, in the respective steps S1) to S6) of the method according to the present invention, the resulting liquid mixture comprising any of compounds VI, V, IV, III or II can either be used directly for the next step (in particular the liquid mixture comprising the compound of formula V resulting from step S2)), or the resulting liquid mixture comprising any of compounds VI, V, IV, III, II or I can be further worked-up by e.g. (i) exchange of the one or more non-halogenated organic solvents from the liquid reaction medium for one or more other non-halogenated organic solvents (in particular the liquid mixture comprising the compound of formula IV resulting from step S3)), or by isolating the respective compound formed in a respective step from the resulting liquid mixture (in particular the compounds of formulae VI, III, II and I), as is explained in more detail below.

Conducting or carrying out step S2) of the method according to the present invention in a liquid reaction medium comprising or consisting of the one or more non-halogenated (preferably polar-aprotic, non-aromatic) organic solvents as defined above, preferably in N,N-dimethyl formamide, is particularly beneficial, as it allows using liquid reaction media comprising the same one or more non-halogenated organic solvents (as is described in more detail below) for conducting steps S2), S3) and S4) (see below for details).

The present inventors have further found that using a phase transfer catalyst (for preferred phase transfer catalysts see below) in step S2) allows to carry out the reaction of step S2) at comparatively low temperatures, preferably at a temperature not exceeding 40 °C, more preferably not exceeding 30 °C, at which temperature(s) an undesired side reaction does not occur or does only occur to a negligible extent, between (i) the agent suitable for introducing the protective group PG which can be removed by hydrogenolysis, in particular benzyl chloride, benzyl bromide and mixtures thereof, and (ii) the base which is present in the liquid reaction medium of step S2) (in particular K₂CO₃). Another advantage of carrying out step S2) in a liquid reaction medium as defined above and at a temperature not exceeding 30 °C is, that epimerization at the carbon atom carrying the acetyl group of the compound of the formula V (carbon ring atom No. 7, "C7") does not occur or does only occur to a negligible extent.

Other methods of making a compound of formula V are e.g. known from EP 2 332 410 A1 or CZ300995 B6.

Preferred is a method of making according to the present invention as defined herein (or a method of making according to the present invention as described herein as being preferred), wherein
- the liquid reaction medium used in step S2) comprises the one or more non-halogenated organic solvents, preferably comprises N,N-dimethyl formamide, in a total amount of ≥ 90 wt.-%, preferably of ≥ 95 wt.-%, relative to the total weight of the liquid reaction medium;
   and/or
- the one or at least one of the more non-halogenated organic solvents is N,N-dimethyl formamide;
   and/or
- the base has a pK_{b} at 25 °C of ≤ 4 and/or is selected from the group consisting of alkali metal carbonates, earth alkali carbonates and mixtures thereof, preferably the base comprises or is K₂CO₃;
   and/or
- reacting in step S2) the compound of formula VI with the agent suitable for introducing, to the free hydroxyl group of the compound of formula VI, a protective group PG which can be removed by hydrogenolysis, is carried out at a temperature not exceeding 40 °C, more preferably at a temperature not exceeding 30 °C;
   and/or
- the phase transfer catalyst comprises or is a quarternary ammonium salt (preferably a quarternary ammonium halogenide salt), preferably selected from the group consisting of tetrabutyl ammonium chloride, tetrabutyl ammonium bromide, tetrabutyl ammonium iodide, benzyltriethylammonium chloride; benzyltriethylammonium bromide, benzyltriethylammonium iodide and mixtures thereof, more preferably the phase transfer catalyst comprises or is tetrabutyl ammonium bromide;
   and/or
- the compound of formula VI is reacted with an agent suitable for introducing a protective group PG selected from the group consisting of benzyl, p-methoxybenzyl, 3,4-dimethoxybenzyl, (4-methoxyphenyl)diphenylmethyl and triphenylmethyl, preferably wherein the compound of formula VI is reacted with an agent suitable for introducing a benzyl protective group, preferably selected from the group consisting of benzyl chloride, benzyl bromide and mixtures thereof (even more preferred wherein the compound of formula VI is reacted with benzyl bromide);
   and/or
- the protective group PG in the compound of formula V is a protective group which can be removed by hydrogenolysis, preferably PG is a protective group selected from the group consisting of benzyl, p-methoxybenzyl, 3,4-dimethoxybenzyl, (4-methoxyphenyl)diphenylmethyl and triphenylmethyl, more preferably PG is a benzyl protective group.

Also preferred is a method of making according to the present invention as defined herein (or a method of making according to the present invention as described herein as being preferred), wherein the compound of formula VI is reacted with an agent suitable for introducing a protective group PG, preferably selected from the group consisting of benzyl chloride, benzyl bromide and mixtures thereof, and wherein any excess agent suitable for introducing the protective group PG is decomposed by heating to a temperature in the range of 50 to 90 °C, preferably of 60 to 80 °C, in the presence of the base, once the compound of formula V has formed (preferably once the formation of the compound of formula V is completed in step S2) and before any step S3) is carried out).

For example, once the compound of formula V has formed in step S2), the resulting mixture may be heated to a temperature of about 70 °C to facilitate the decomposition or inactivation of any present excess benzyl bromide or benzyl chloride, probably by reaction with the base, which is also present in the mixture. The so-formed liquid mixture (after removal of any solid substances by filtration) may be directly used for the next reaction step S3) without further treatment of the resulting mixture, isolation of the compound of formula V, or work-up. In this preferred variant of the method of the present invention, no aqueous work-up (i.e. no further treatment steps involving the addition of water) is necessary. It was found by the present inventors that abstaining from further steps of aqueous work-up can further reduce the extent of epimerization otherwise occurring at the carbon atom carrying the acetyl group of the compound of the formula V ("C7"). In addition, directly using the liquid mixture resulting from step S2) (as explained above) has economic and ecological advantage compared to a work-up, in particular an aqueous work-up, of the liquid mixture.

A particular advantage of the preferred variant of the method according to the present invention, wherein any excess agent suitable for introducing the protective group PG is decomposed by heating to a temperature in the range of 50 to 90 °C in the presence of the base (as defined above) is realized, when the liquid mixture formed in step S2) (after decomposition or inactivation of any present excess benzyl bromide or benzyl chloride and preferably after removal of any solid substances by filtration, see above) is directly used for the reaction in step S3), preferably without prior isolation or purification of the compound of formula V obtained in step S2) (see below for details): By removal of any excess benzyl bromide or benzyl chloride from the mixture received after step S2), an undesired side reaction in step S3) is avoided or reduced to the largest extent possible, which may otherwise have occurred between the excess benzyl bromide or benzyl chloride still present and the de-methylated amine function of the compound of formula IV as formed in step S3) (as shown below). Such undesired side reaction may, for example, produce the undesired side product of the compound of formula X as shown below:

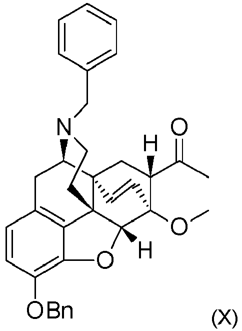

Furthermore preferred is a method of making according to the present invention as defined herein (or a method of making according to the present invention as described herein as being preferred), wherein the method further comprises the additional step:
S3) reacting a compound of formula V as defined in step S2) above, preferably as received in step S2), wherein PG is a protective group which can be removed by hydrogenolysis, preferably wherein PG is a protective group selected from the group consisting of benzyl, p-methoxybenzyl, 3,4-dimethoxybenzyl, (4-methoxyphenyl)diphenylmethyl and triphenylmethyl, more preferably wherein PG is a benzyl protective group,
in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium;
   wherein preferably the one or more non-halogenated organic solvents are selected from the group consisting of N,N-dimethyl formamide, γ-butyrolactone, N-methyl-2-pyrrolidone, N-butyl-2-pyrrolidone and dihydrolevoglucosenon, more preferably wherein the one or at least one of the more non-halogenated organic solvents is N,N-dimethyl formamide;
with a de-methylating agent, to receive a compound of formula IV 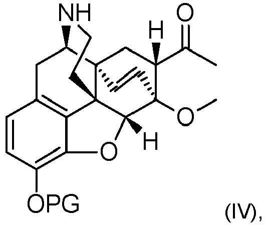
wherein PG has the above meaning.

A method of making according to the present invention as defined herein (or a method of making according to the present invention as described herein as being preferred) is also preferred, wherein the liquid mixture comprising the compound of formula V as received after step S2)) is directly used for the reaction in step S3), preferably without prior isolation or purification (preferably with the exception of prior removal of any solid substances by filtration) of the compound of formula V obtained in step S2).

As was already explained above, said direct use of the liquid mixture comprising the compound of formula V as received after step S2)) for the reaction in step S3) offers the greatest advantages when said direct use is combined with the preferred variant of the method according to the present invention, wherein (once the compound of formula V has formed in step S2), see above) any excess agent suitable for introducing the protective group PG (in particular any benzyl bromide and/or benzyl chloride) was previously decomposed by heating to a temperature in the range of 50 to 90 °C in the presence of the base.

In a preferred variant of step S3) as described here above, the compound of formula IV is not isolated from the liquid mixture received after step S3), but any solvents or reagents used during the work-up of the liquid mixture comprising the compound of formula IV as received after step S3) are preferably removed or are preferably exchanged for the one or more non-halogenated organic solvents which are to be used in a subsequent step S4) (see below for details).

Other methods of making compounds of formula IV are e.g. known from EP 2 332 410 A1.

Likewise preferred is a method of making according to the present invention as defined herein (or a method of making according to the present invention as described herein as being preferred), wherein the method further comprises the additional step:
S4) reacting a compound of formula IV as defined in step S3) above, preferably as received in step S3),
in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium,
   wherein preferably the one or more non-halogenated organic solvents are selected from the group consisting of N,N-dimethyl formamide, γ-butyrolactone, N-methyl-2-pyrrolidone, N-butyl-2-pyrrolidone and dihydrolevoglucosenon, more preferably wherein the one or at least one of the more non-halogenated organic solvents is N,N-dimethyl formamide;
with a halogeno-methylcyclopropane (preferably selected from chloromethyl cyclopropane, bromomethyl cyclopropane and mixtures thereof, more preferably with bromomethyl cyclopropane), to receive a compound of formula III 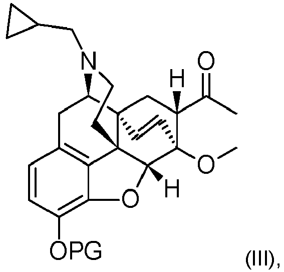
wherein PG is a protective group which can be removed by hydrogenolysis, preferably wherein PG is a protective group selected from the group consisting of benzyl, p-methoxybenzyl, 3,4-dimethoxybenzyl, (4-methoxyphenyl)diphenylmethyl and triphenylmethyl, more preferably wherein PG is a benzyl protective group.

A particular advantage of step S4) of the method of the present invention is, that the compound of formula III can be easily isolated from the liquid mixture resulting from step S4) by crystallization. Crystallization can preferably be done by techniques known in the technical field, preferably by increasing the polarity of the respective liquid mixture, preferably by adding water to said liquid mixture, and cooling the respective liquid mixture. Moreover, the present inventors have found that surprisingly no solvates were formed of the compound of formula III as received from step S4) of the method according to the present invention.

Other methods of making compounds of formula III are e.g. known from EP 2 332 410 A1.

In a preferred variant of the method according to the present invention, said method comprises the reactions of step S3) and of step S4), wherein both steps are carried out in liquid reaction media comprising the same one or more non-halogenated organic solvents, as is further explained below.

Also preferred is a method of making according to the present invention as defined herein (or a method of making according to the present invention as described herein as being preferred), wherein the method further comprises the additional step:
S1) providing or preparing oripavine of formula VII 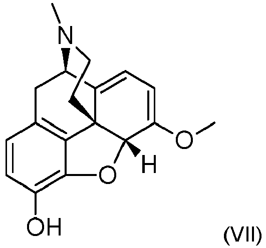
and reacting the compound of formula VII
in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium;
   wherein preferably the one or at least one of the more non-halogenated organic solvents is ethanol;
with 3-buten-2-one under conditions of a Diels-Alder reaction, preferably under conditions of temperature and pressure so that boiling of the liquid reaction medium is avoided,
to receive a compound of formula VI as defined in step S2) above.

Oripavine is the major alkaloid of *Papaver orientale* and can be isolated therefrom, as well as from *Papaver bracteatum* by methods known in the technical field concerned.

In a preferred variant of the method according to the present invention, the one or more non-halogenated organic solvents present in the liquid reaction medium used in step S1) comprises or is ethanol. Reacting the compound of formula VII with 3-buten-2-one under conditions of a Diels-Alder reaction in step S1) can preferably also be carried out in a liquid reaction medium comprising more than one non-halogenated organic solvents, preferably comprising or consisting of ethanol and toluene, where preferably the amount of toluene used does not exceed 5 wt.-% of the total weight of the non-halogenated organic solvents present in the liquid reaction medium.

In variants of step S1) of the method according to the present invention, a reaction temperature not exceeding 80 °C may be selected and an increased pressure in the range of from 1 barg (100 hPa (gauge)) to 3 barg (300 hPa (gauge)) may be applied.

It has been found by the present inventors that the compound of formula VI as received in step S1) of the method of making of the present invention, including the preferred variants of step S1), only comprises a very low amount of its C7-epimer. Said very low amount of C7-epimer can be further reduced by common purification methods, preferably by crystallization.

When a liquid reaction medium comprising the same non-halogenated organic solvent or solvents (as is used in step S1)) is also used in other steps of the method according to the present invention, e.g. in step S6) (as is described in more detail below), a further advantage of the method according to the present invention is, that the overall number of different solvents required for the present method is reduced.

Other methods of making the compound of formula VI are e.g. known from document EP 2 332 410 A1.

It is then also preferred a method of making according to the present invention as defined herein (or a method of making according to the present invention as described herein as being preferred), wherein the method further comprises the additional step:
S5) reacting a compound of formula III as defined in step S4) above, preferably as received in step S4),
in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium;
   wherein preferably the one or more non-halogenated organic solvents are selected from the group of dialkylethers,
   and/or are selected from the group consisting of methyl tert.-butyl ether; diethyl ether; 1,4-dioxan and tetrahydrofuran, preferably are selected from the group consisting of methyl tert.-butyl ether; diethyl ether and 1 ,4-dioxan, more preferably wherein the one or at least one of the more non-halogenated organic solvents is methyl tert.-butyl ether;
with a tert.-butyl magnesium halide, preferably tert.-butyl magnesium chloride, under conditions of a Grignard reaction, to receive a compound of formula II

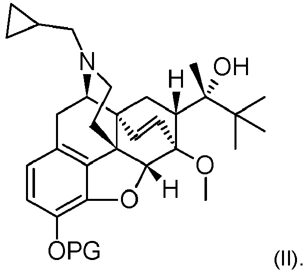

Other methods of making a compound of formula II are e.g. known from EP 2 332 410 A1.

Also preferred is a method of making according to the present invention as defined herein (or a method of making according to the present invention as described herein as being preferred), wherein the method further comprises the additional step
S6) reacting a compound of formula II as defined in step S5) above, preferably as received in step S5),
in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium;
   wherein preferably the one or at least one of the more non-halogenated organic solvents is ethanol;
under conditions suitable for removing the protective group PG by hydrogenolysis,,
to receive buprenorphine of formula I

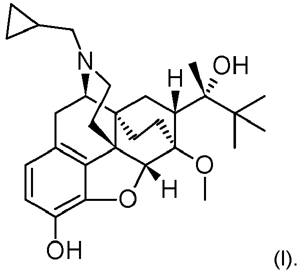

In a preferred variant of the method according to the present invention, the one or more non-halogenated organic solvents of the liquid reaction medium used in step S6) comprises or is ethanol (where preferably ethanol is used in a total amount of ≥ 85 wt.-%, preferably of ≥ 95 wt.-%, relative to the total weight of the liquid reaction medium in step S6)), as is also described below. When a liquid reaction medium comprising the same non-halogenated organic solvent or solvents (as used in step S6)) is also used in other steps of the method according to the present invention, e.g. in step S1) (as is described in more detail above and below), a further advantage of the present preferred variant of the method according to the present invention is, that the overall number of different solvents required for the present method is reduced.

The inventors have found that it is preferred in the hydrogenation reaction of step S6) to select a temperature not exceeding 30 °C and to apply an increased hydrogen pressure in the range of from 5 barg (0.5 MPa (gauge)) to 30 barg (3 MPa (gauge)), preferably of from 5 barg (0.5 MPa (gauge)) to 15 barg (1.5 MPa (gauge)). Under these reaction conditions (in particular where the reaction temperature does not exceed 30 °C), a reduced formation of side-products was observed. The inventors have further found in own experiments, that it is preferred in said preferred variant of the method according to the present invention to use a heterogenous noble metal hydrogenation catalyst, preferably a supported palladium catalyst, more preferably palladium on an activated carbon support ("Pd/C").

In some cases, it may be favourable, once hydrogenolysis in step S6) is completed, to further treat the resulting liquid mixture with another reducing agent, preferably selected from the group consisting of LiAlH₄, NaBH₄, LiBH₄, lithium tri-sec-butylborohydride (L-Se-lectride^{®}), sodium-bis(2-methoxyethoxy)-aluminiumhydride (Red-Al^{®}) and diisobutylalu-miniumhydride (DiBAIH). It has also been found that, by this additional treatment, the amount of undesired side-products which may have formed in step S6) can be substantially reduced.

The compound of formula I as received from the method of making according to the present invention as described herein can further be purified, preferably by recrystallization as is known in the technical field, preferably from a mixture of ethanol and water.

The compound of formula I as received from the method of making according to the present invention as described herein, or the recrystallized compound of formula I as described above, can further be converted to its hydrochloride salt as is known in the technical field, preferably by reacting said compound of formula I with aqueous hydrochloric acid in a lower alkyl alcohol, preferably in ethanol.

Other methods of making a compound of formula I are e.g. known from EP 2 332 410 A1.

In addition, there is also preferred a method of making according to the present invention as defined herein (or a method of making according to the present invention as described herein as being preferred), wherein the reaction in each of the steps S1), S2), S3), S4), S5) and S6) is carried out in a liquid reaction medium comprising one or more non-halogenated organic solvents, in a total amount of ≥ 85 wt.-%, preferably of ≥ 90 wt.-%, more preferably of ≥ 95 wt.-%, even more preferably of ≥ 98 wt.-%, relative to the total weight of the liquid reaction medium.

Moreover, a method of making according to the present invention as defined herein (or a method of making according to the present invention as described herein as being preferred) is preferred, wherein
the reaction in each of the steps S2) and S3), or in each of the steps S2), S3) and S4), is carried out in a liquid reaction medium comprising one or more non-halogenated organic solvents in a total amount of ≥ 85 wt.-%, preferably of ≥ 90 wt.-%, and still more preferably of ≥ 95 wt.-%, relative to the total weight of the liquid reaction medium,
   and
the one or more non-halogenated organic solvents are selected in each case from the group consisting of N,N-dimethyl formamide, γ-butyrolactone, N-methyl-2-pyrrolidone, N-butyl-2-pyrrolidone and dihydrolevoglucosenon,
   preferably wherein the reaction in each of the steps S2) and S3), or in each of the steps S2), S3) and S4), is carried out in a liquid reaction medium wherein at least one of the one or more non-halogenated organic solvents (of the one or more non-halogenated organic solvents which are selected in each case from the group consisting of N,N-dimethyl formamide, γ-butyrolactone, N-methyl-2-pyrrolidone, N-butyl-2-pyrrolidone and dihydrolevoglucosenon) present in the liquid reaction medium is the same in each of the steps S2) and S3), or in each of the steps S2), S3) and S4), and is present (in each case) in a total amount of ≥ 85 wt.-%, preferably of ≥ 90 wt.-%, more preferably of ≥ 95 wt.-%, relative to the total weight of the totality of non-halogenated organic solvents of the liquid reaction medium in the respective step S2), S3) and/or S4);

In other preferred alternatives of the method according to the present invention:
- the reaction in each of the steps S2), S3) and S4) is carried out in a liquid reaction medium comprising the same one or more non-halogenated organic solvents, more preferably wherein the reaction in each of the steps S2), S3) and S4) is carried out in a liquid reaction medium comprising N,N-dimethyl formamide in a total amount of ≥ 85 wt.-%, preferably of ≥ 90 wt.-%, more preferably of ≥ 95 wt.-%, relative to the total weight of the liquid reaction medium;
   and/or
- wherein the reaction in each of the steps S1) and S6) is carried out in a liquid reaction medium comprising ethanol in a total amount of ≥ 85 wt.-%, preferably of ≥ 90 wt.-%, more preferably of ≥ 95 wt.-%, relative to the total weight of the liquid reaction medium;
   and/or
- the method comprises steps S1), S2), S3), S4) and S6), and not more than two different solvents are used in said method, where all of said different solvents are non-halogenated organic solvents (preferably ethanol in steps S1) and S6) and N,N-dimethyl formamide in steps S2), S3) and S4)).

In a particularly preferred variant of the method according to the present invention, the reaction in each of the steps S1), S2), S3), S4), S5) and S6) is carried out in a liquid reaction medium comprising no halogenated organic solvents, i.e. no halogenated organic solvents are used in this particularly preferred variant of the method according to the present invention.

As used herein, the expression that "at least one of the one or more non-halogenated organic solvents present in the liquid reaction medium is the same" in each of certain steps (e.g. in steps S3) and S4)) of the method according to the present invention means, that in each of said certain steps the same non-halogenated organic solvent is present in the liquid reaction media (e.g. N,N-dimethyl formamide is present in the liquid reaction media of step S3) and step S4)), however said same non-halogenated organic solvents (as e.g. used in steps S3) and S4)) are not necessarily identical, neither in quantity, nor in the ratio of the respective non-halogenated organic solvent to any other solvents potentially present in said liquid reaction media.

Preferred is then also a method of making according to the present invention as defined herein (or a method of making according to the present invention as described herein as being preferred), wherein the de-methylating agent used in step S3) comprises or is an azodicarboxylic acid alkyl ester of formula IX

R¹-OOC-N=N-COOR² (IX),

wherein R¹ and R² are independently selected from the group consisting of linear alkyl with 1 to 6 carbon atoms and branched alkyl with 1 to 6 carbon atoms, preferably selected from the group consisting of methyl, ethyl, n-propyl, isopropyl, n-butyl, sec.-butyl, tert.-butyl, pentyl and hexyl, wherein more preferably the azodicarboxylic acid alkyl ester of formula IX comprises or is diisopropyl azodicarboxylate,
preferably wherein the de-methylating agent used in step S3) comprises or is diisopropyl azodicarboxylate and the one or at least one of the more non-halogenated organic solvents used in the liquid reaction medium in step S3) comprises or is dimethyl formamide,
more preferably wherein the de-methylating agent used in step S3) comprises or is diisopropyl azodicarboxylate and the reaction in step S3) is carried out in a liquid reaction medium comprising ≥ 90 wt.-%, preferably ≥ 95 wt.-%, of N,N-dimethyl formamide, relative to the total weight of the liquid reaction medium.

It has been found by the present inventors that using an azodicarboxylic acid alkyl ester of formula IX as de-methylating agent in step S3) allows to carry out said step S3) in a liquid reaction medium comprising one or more non-halogenated organic solvents, preferably selected rom the group consisting of N,N-dimethylformamide, γ-butyrolactone, N-methyl-2-pyrrolidone, N-butyl-2-pyrrolidone and dihydrolevoglucosenon (and more preferably in N,N-dimethylformamide, as defined above). The possibility of carrying out said step S3) in a liquid reaction medium comprising one or more non-halogenated organic solvents (specifically as defined here above) then further allowed to set up the method of making according to the present invention in such a way that the sequence of reaction steps S2), S3) and S4) can be carried out in liquid reaction media comprising non-halogenated organic solvent or solvents in the amounts or preferred total amounts as defined above, wherein at least one of the one or more non-halogenated organic solvents present in the liquid reaction medium is the same in each of the steps S2), S3) and S4), and is preferably present in a total amount as specified above, thereby increasing or further increasing efficiency and sustainability of the method according to the present invention.

In a further variant, a method of making according to the present invention as defined herein is preferred (or a method of making according to the present invention as described herein as being preferred), wherein the reaction in each of the steps S1) and S6) is carried out in a liquid reaction medium comprising ethanol, in a total amount of ≥ 85 wt.-%, preferably of ≥ 90 wt.-%, more preferably of ≥ 95 wt.-%, relative to the total weight of the liquid reaction medium.

It has been found by the present inventors that step S1) of the present method can beneficially be carried out in a liquid reaction medium comprising ethanol in the total amount or preferred total amounts as specified here above. Carrying out said step S1) in such liquid reaction medium comprising ethanol (and preferably under the preferred conditions as described above) then further allowed to set up the method of making according to the present invention in such a way that liquid reaction media comprising the same non-halogenated organic solvent or solvents can be used in reaction steps S1) and S6), thereby further reducing the overall number of different solvents required for the present method and thus further increasing its efficiency.

As is explained above, reducing the overall number of solvents used in the method according to the present invention and/or reducing or avoiding the use of halogenated organic solvents makes the present method of making better suited for industrial scale.

A method of making according to the present invention as defined herein (or a method of making according to the present invention as described herein as being preferred) is also preferred, wherein
- the protective group PG in each of compounds of formula II, III, IV and V is the same and is selected from the group consisting of benzyl, p-methoxybenzyl, 3,4-dimethoxybenzyl, (4-methoxyphenyl)diphenylmethyl and triphenylmethyl, preferably wherein the protective group PG in each of compounds of formula II, III, IV and V is benzyl;
   and/or
- the method comprises at least steps S2), S3) and preferably S4);
   and/or
- the method is a method of making buprenorphine from oripavine, or the method is part of a method of making buprenorphine from oripavine.

A particularly preferred variant of the method of making according to the present invention as defined herein (or a method of making according to the present invention as described herein as being preferred) is also preferred, wherein the method is a method of making buprenorphine from oripavine, comprising the following steps or sequence of steps:
S1) providing or preparing oripavine of formula VII 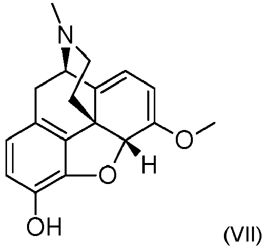
   and reacting the compound of formula VII
   with 3-buten-2-one under conditions of a Diels-Alder reaction, preferably under conditions of temperature and pressure so that boiling of the liquid reaction medium is avoided,
   to receive a compound of formula VI 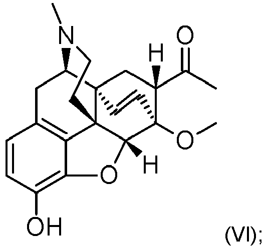
S2) reacting the compound of formula VI as received in step S1)
   at a temperature not exceeding 30 °C, in the presence of a base, preferably wherein the base has a pK_{b} at 25 °C of ≤ 4 and/or is selected from the group consisting of alkali metal carbonates, earth alkali carbonates and mixtures thereof (more preferably wherein the base comprises or is K₂CO₃); and
   in the presence of a phase transfer catalyst, preferably wherein the phase transfer catalyst comprises or is a quarternary ammonium salt (preferably a quarternary ammonium halogenide salt),
   with an agent suitable for introducing a protective group PG which can be removed by hydrogenolysis, preferably with an agent suitable for introducing a benzyl protective group PG,
   to receive a compound of formula V 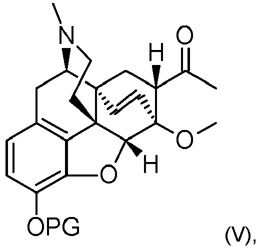
   wherein PG is a protective group which can be removed by hydrogenolysis, preferably wherein PG is benzyl;
S3) reacting the compound of formula V as received in step S2) with a de-methylating agent, preferably with an azodicarboxylic acid alkyl ester of formula IX

   R¹-OOC-N=N-COOR² (IX),

   wherein R¹ and R² are independently selected from linear or branched alkyl with 1 to 6 carbon atoms, preferably with diisopropyl azodicarboxylate,
   to receive a compound of formula IV 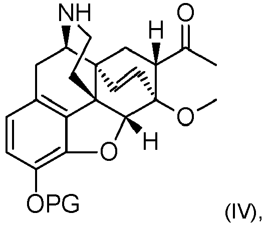
   wherein PG has the above meaning;
S4) reacting the compound of formula IV as received in step S3) with a halogeno-methylcyclopropane, preferably with bromomethyl cyclopropane, to receive a compound of formula III 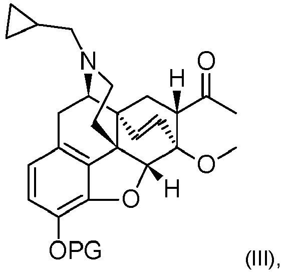 wherein PG has the above meaning;
S5) reacting the compound of formula III as received in step S4) with a tert.-butyl magnesium halide, preferably with tert.-butyl magnesium chloride, under conditions of a Grignard reaction, to receive a compound of formula II 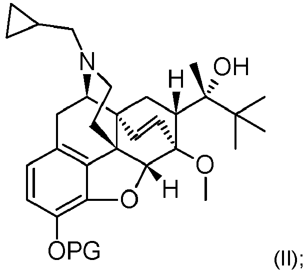 and
S6) reacting the compound of formula II as received in step S5) under conditions suitable to remove the protective group PG by hydrogenolysis,
   to receive buprenorphine of formula I 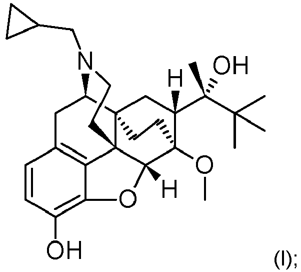 wherein
   - each of the steps S1), S2), S3), S4), S5) and S6) is carried out in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium,
      and/or
   - the reaction in each of the steps S2), S3) and S4) is carried out in a liquid reaction medium comprising one or more non-halogenated organic solvents, selected in each case from the group consisting of N,N-dimethyl formamide, γ-butyrolactone, N-methyl-2-pyrrolidone, N-butyl-2-pyrrolidone and dihydrolevoglucosenon,
      preferably wherein the reaction in each of the steps S2) and S3), or in each of the steps S2), S3) and S4), is carried out in a liquid reaction medium comprising the same one or more non-halogenated organic solvents, more preferably wherein the reaction in each of the steps S2), S3) and S4) is carried out in a liquid reaction medium comprising N,N-dimethyl formamide in a total amount of ≥ 85 wt.-%, preferably of ≥ 90 wt.-%, more preferably of ≥ 95 wt.-%, relative to the total weight of the liquid reaction medium;
      and/or
   - wherein the reaction in each of the steps S1) and S6) is carried out in a liquid reaction medium comprising ethanol in a total amount of ≥ 85 wt.-%, preferably of ≥ 90 wt.-%, more preferably of ≥ 95 wt.-%, relative to the total weight of the liquid reaction medium.

Where the method according to the present invention is a method of making buprenorphine from oripavine as defined here above, all aspects of the different steps S1), S2), S3), S4), S5) and S6) as discussed herein also apply *mutatis mutandis* to the method according to the present invention which is a method of making buprenorphine from oripavine (comprising all of the steps S1), S2), S3), S4), S5) and S6)), and *vice versa.*

### Examples:

The following examples are meant to further explain and illustrate the present invention, without limiting its scope.

All amounts given as "molar equivalents" in the present examples are calculated relative to the molar amount of the respective starting compound or starting material used, unless otherwise indicated.

### Example 1: Synthesis of 7α-acetyl-6,7,8,14-tetrahydro-6,14-endo-ethenooripavine ("Etheno-oripavine", compound of formula VI) from oripavine (Step S1))

A pressure reactor was charged with oripavine (50 g, 168 mmol) and ethanol (275 mL). To the resulting suspension, 2.2 molar equivalents (relative to the molar amount of starting compound oripavine) of 3-buten-2-one (synonym: methyl vinyl ketone, 25.9 g) were added under stirring at room temperature. The resulting suspension was heated to and kept at 80 °C and stirred under pressure (not exceeding 2.5 barg) for 26 hrs. The resulting mixture was cooled to 70 °C, followed by pressure release (to normal pressure). The reaction mixture was subsequently cooled to 25 °C, then further to 0 °C and stirred at 0 °C for another 2 hrs. The resulting solid product was filtered, washed with two times 25 mL ethanol and dried under vacuum. Etheno-oripavine was obtained as a white to brownish crystalline solid (52.5 g, 85% yield).

### Example 2: Synthesis of 7α-acetyl-3-benzyl-6.7,8,14-tetrahydro-6,14-endo-ethenooripav-ine ("Etheno-benzoripavine", compound of formula V) (Step S2))

Etheno-oripavine (52.5 g, 134 mmol; synthesis as described in example 1 above), tetrabutylammonium bromide (0.05 molar equivalents) and benzyl bromide (1.2 molar equivalents) were suspended in N,N-dimethyl formamide ("DMF", 157.5 mL) at 25 °C and stirred for 10 min. Potassium carbonate (1.5 molar equivalents) was added and the suspension was stirred at 25 °C for 5 hrs. The reaction mixture was then heated to 70 °C, stirred at 70 °C for 4 hrs and cooled to 25 °C. The resulting mixture was filtered and the filter cake rinsed with DMF (52.5 mL). Etheno-benzoripavine was obtained as an about 25%-w/w solution in DMF, which was used in the next step (step S3)) without further isolation or purification.

### Example 3: Synthesis of 7α-acetyl-3-benzyl-6,7,8,14-tetrahydro-6,14-endo-ethenonoror-ipavine ("Etheno-nororipavine", compound of formula IV) (Step S3))

A reactor was charged with the resulting solution of etheno-benzoripavine in DMF (about 130 mmol) as received from example 2 above, butylated hydroxytoluene (BHT, 0.2 molar equivalents) and the resulting mixture was heated to 55 °C. Diisopropyl azodicarboxylate (DIAD, 2.05 molar equivalents) was added over 6 hrs at 55 °C and the resulting mixture was stirred at 55 °C for further 6 hrs.

The reaction mixture was then cooled to 25 °C and dimedone (3.05 molar equivalents) and methanol (2.3 molar equivalents) were added. The mixture was then cooled and filtered. The filtered precipitate was washed with cold DMF (25 mL) and the combined filtrates were slowly quenched by pouring onto a 0 °C cold, stirred mixture of toluene (210 mL), deionized water (140 mL) and 32% HCl (1.12 molar equivalents). After the addition was complete, the resulting two-phasic mixture was heated to 25 °C and stirred for further 30 min. The product-carrying aqueous phase was collected and the organic phase was extracted with a mixture of deionized water (100 mL) and 32% HCl (0.07 molar equivalents). Toluene (210 mL) was added to the combined aqueous phases, the resulting mixture was cooled to 0 °C and its pH was adjusted to pH 11 with 30% aqueous NaOH. After the pH was adjusted, the mixture was stirred for further 30 min. and the phases were separated. The aqueous phase was extracted with toluene (100 mL) and the product-carrying organic phases were combined.

For exchanging the solvent(s) of the combined organic phases (mainly comprising toluene and DMF before the solvent exchange), the combined organic phases were first concentrated by vacuum distillation until about 270 mL of their solvents had been distilled off. The, residue was filled up with DMF to a target level of 170 mL and the resulting mixture was heated to 55 °C. An approximately 25-30%-w/w solution of etheno-nororipavine in DMF was obtained, which was used in the next step (step S4)) without further isolation or purification.

### Example 4: Synthesis of 7α-acetyl-3-benzyl-17-cyclopropylmethyl-6,7,8,14-tetrahydro-6,14-endo-ethenonororipavine ("Desbutyl-buprenorphene", compound of formula III) (Step S4))

To the resulting solution of etheno-nororipavine in DMF as received from example 3 above, sodium carbonate (1.2 molar equivalents, relative to 134 mmol of etheno-oripavine, starting compound from step S2)), butylated hydroxytoluene (BHT, 0.05 molar equivalents) and bromomethyl cyclopropane (1.05 molar equivalents) were added at 55 °C and the reaction mixture was stirred at 55 °C for 10 hrs. The resulting suspension was heated to 70 °C and filtered at this temperature. Reactor and filter cake were washed with 25 ml DMF. DMF was added to dilute the mixture until the target level of about 26%-w/w was reached. The resulting mixture was then cooled to 65 °C and deionized water (10 mL) was added at 65 °C over 1 hour. The mixture was cooled to 60 °C and stirred for further 30 min. The resulting supersaturated solution was seeded with 0.12 %-w/w of (previously produced) desbutyl-buprenorphene and stirred for 45 minutes. The mixture was cooled to 30 °C within 3 hrs. and subsequently further to 0 °C within 1 h. At 0 °C, water (25 mL) was added within 1 hour. The resulting suspension was stirred at 0 °C for one further hour and the resulting solid product was isolated by filtration. The filter cake was washed twice with 52.5 mL of a mixture of DMF/water (2 : 1, v/v), where each portion had been pre-cooled to 0 °C, followed by two washes with water (50 mL each portion, 25 °C). The resulting wet product was dried for 25 hrs. at 50 °C in vacuo. The dried desbutyl-buprenorphene was obtained as a white to slightly yellowish crystalline solid (53.3 g, 107 mmol, 80% yield over 3 steps).

### Example 5: Synthesis of 3-benzyl-17-cyclopropylmethyl-6,14-endo-etheno-7a-(2-hydroxy-3,3-dimethyl-2-butyl)-6,7,8,14-tetrahydronoraripavine ("Benz-buprenorphene"), compound of formula II) (Step S5))

Preparation of a Grignard reagent: Methyl-tert.-butyl ether (MTBE, 300 g) was added to a reactor. Then, tert.-butyl magnesium chloride (25% in diethylether, 6 molar equivalents) was added to form a white/gray suspension. The diethyl etherwas distilled off, the resulting mixture was cooled to 15 °C and filled up with MTBE (400 g) to a final volume of 500 mL.

In a separate reactor, desbutyl-buprenorphene (50.0 g, as received from example 4 above) was dissolved in 1,4-dioxane (150 g) at 25 °C to receive a desbutyl-buprenorphene-solution.

The desbutyl-buprenorphene-solution (see above) was dosed onto the Grignard reagent (see above) within 1 h in such a way that a temperature of 25 °C was not exceeded. Once dosing was complete, the reactor from which the desbutyl-buprenorphene solution was withdrawn was washed with MTBE (50 mL) and the reaction mixture was stirred for one hour at 15 °C. Subsequently, the resulting mixture was quenched within 1 h to a solution of ammonium chloride (18.2 molar equivalents, relative to the molar amount of desbutyl-buprenorphene used) and water (450 mL) cooled to 5 °C. After finished addition, the two-phase mixture was stirred for 1 h at 25 °C and the phases were separated and left standing for 30 min. The solvent of the product-containing organic phase was then exchanged for ethanol and the filling level was adjusted to 400 mL.

The resulting ethanol mixture was seeded with previously produced benz-buprenorphene (0.12 % w/w, relative to the total weight of desbutyl-buprenorphene used) and stirred for 1 h. The mixture was cooled to 25 °C within 3 hrs. At 25 °C, water (100 mL) was added over 2 hrs., then the resulting mixture was cooled to 0 °C within 1.5 hrs. The thickened suspension received was stirred for another 4 hrs. at 0 °C and then filtered off. The reactor and the filter cake were both washed with 100 g of a 0 °C cool mixture of ethanol / water 2:1 (w/w), then the filter cake was washed with water (100 mL). The resulting wet product was dried in a drying oven for 24 hrs. at 50 °C and 100 mbar (i.e. 100 hPa). The dried benz-buprenorphene was obtained as a white, fluffy solid (53.5 g, 96 mmol).

### Example 6: Synthesis of buprenorphine (compound of formula I) (Step S6))

In a hydrogenation apparatus, benz-buprenorphene (as received from example 5 above, 50.0 g) and a hydrogenation catalyst (Pd/C Noblyst P1052, 12 %-w/w, relative to the amount of benz-buprenorphene used) were suspended in 650 mL ethanol. After atmospheric exchange to H₂ (3 x 5 barg, corresponding to 3 x 0.5 MPa (gauge)) at 20 °C, the pressure is adjusted to 15 barg (corresponding to 1.5 MPa (gauge)) at 20 °C. The mixture was then heated to 28 °C under stirring within 30 min. and subsequently hydrogenated at that temperature for 30 hrs. After depressurization and inertization the catalyst was filtered off and the filter cake rinsed with 140 mL ethanol. The resulting ethanol mixture was concentrated under normal pressure and subsequently cooled to 60 °C, to a target volume of about 500 mL.

At 60 °C, 100 mL deionized water were then added within 15 min. Afterwards, the resulting mixture was seeded with 0.5%-w/w buprenorphine (previously produced, relative to the amount of benz-buprenorphene used) and stirred for 10 min at 60 °C. Further 200 mL of deionized water were added at 60 °C over 60 min, the resulting suspension was cooled to 0°C within 4 hrs. and stirred for an additional hour. The resulting product was isolated by filtration at 0 °C and washed with 2 x 50 g of a pre-cooled mixture of ethanol / deionized water 1:1 (v/v). The wet product was then dried at 35 °C for 16 hrs. at a pressure of < 100 mbar (corresponding to < 100 hPa). 39.6 g dried buprenorphine were obtained as a white, sugar-like solid (purity ≥ 99% by high performance liquid chromatography).

### Example 7: Recrystallization of buprenorphine (compound of formula I)

Buprenorphine as received from example 6 above was recrystallized from ethanol/water to receive a further purified (recrystallized) buprenorphine.

### Example 8: Synthesis of buprenorphine hydrochloride

Recrystallized buprenorphine as received from example 7 above was suspended in methanol and treated with 32-% aqueous hydrochloric acid to receive buprenorphine hydrochloride.

### Example 9: Optimization experiments for Step S2)

In a series of experiments, etheno-benzoripavine was in each case prepared from etheno-oripavine, essentially as described in example 2 above, where, however, the solvent used, the reaction temperature applied and the relative amounts of base and benzyl bromide used were varied as shown in table 1 below.

**Table 1: Variations of reaction conditions in step S2)**

| **Exp.** | **Solvent** | **T [°C]** | **K₂CO₃** | **BnBr** | **Conversion** | **Total impurities*** | **Reaction time** | **PTC** |
|---|---|---|---|---|---|---|---|---|
| 1 | NMP | 70°C | 1.3 eq. | 1.2 eq. | 90.4 % | 6.6 | Overnight | None |
| 2 | NMP | 90°C | 1.3 eq. | 1.2 eq. | 93.6 % | 3.6 | Over night | None |
| 3 | NMP | 70°C | 2.0 eq. | 1.2 eq. | 99.6 % | 1.7 | Over night | None |
| 4 | NMP | 90°C | 2.0 eq. | 1.2 eq. | 99.2 % | 11.7 | Over night | None |
| 5 | DMF | 70°C | 1.3 eq. | 1.2 eq. | 78.6 % | 3.7 | Over night | None |
| 6 | DMF | 90°C | 1.3 eq. | 1.2 eq. | 74.4 % | 8.6 | Over night | None |
| 7 | DMF | 70°C | 2.0 eq. | 1.2 eq. | 99.9 % | 1.5 | Over night | None |
| 8 | DMF | 90°C | 2.0 eq. | 1.2 eq. | 82.9 % | 22.0 | Over night | None |
| 9 | DMF | 70°C | 2.0 eq. | 1.2 eq. | 80 % | 12% epi | Over night | None |
| 10 | DMF | 70°C | 2.0 eq. | 1.2 eq. | 60 % | 20 % epi | 2 hrs | None |
| 11 | DMF | rt | 2.0 eq. | 1.2 eq. | 90% | 10 % Start. cpd./ No epi | 4 hrs | [NBu₄]I 5 mol% |
| 12 | DMF | rt | 2.0 eq. | 1.2 eq. | 90% | 10 % Start. cpd./ No epi | 4 hrs | [NBzEt₃]Cl 5 mol% |
| 13 | DMF | rt | 2.0 eq. | 1.2 eq. | 99.5% | 0,5 % Start. cpd./ No epi | 4 hrs | [NBzEt₃]Br 5 mol% |
| 14 | DMF | rt | 1.1 eq. | 1.05 eq. | 90% | 10 % Start. cpd./ No epi | 3 hrs | [NBu₄]Br 5 mol% |
| 15 | DMF | rt | 1.5 eq. | 1.05 eq. | 97% | 3 % Start. cpd./ No epi | 3 hrs | [NBu₄]Br 5 mol% |
| 16 | DMF | rt | 2.0 eq. | 1.3 eq. | 99% | 1 % epi | 1 h | [NBu₄]Br 5 mol% |
| 17 | DMF | rt | 1.5 eq. | 1.2 eq. | 99.5% | 0,5 % epi | 2 hrs | [NBu₄]Br 5 mol% |
| 18 | DMF | rt | 1.5 eq. | 1.5 eq. | 98% | 2 % epi | 2 hrs | [NBu₄]Br 5 mol% |
| 19 | DMF | rt | 1.5 eq. | 1.2 eq. | 99 % | No epi | 7 hrs | [NBu₄]Br 2 mol% |

| | |
|---|---|
| PTC: | phase transfer catalyst (in mol-% relative to the molar amount of compound of formula VI used in step S2) |
| *: | including epimer (C7) of compound of formula V |
| eq.: | molar equivalents relative to starting compound etheno-oripavine |
| rt: | room temperature |
| BnBr: | benzyl bromide |
| epi: | epimer (C7) of compound of formula V |
| Start. cpd.: | starting compound (etheno-oripavine) |
| NMP: | N-methylpyrrolidone |
| [NBu₄]I: | Tetrabutyl ammonium iodide |
| [NBzEt₃]Cl: | Benzyltriethyl ammonium chloride |
| [NBzEt₃]Br: | Benzyltriethyl ammonium bromide |

As can be seen from table 1 above, using a phase transfer catalyst in a reaction of step S2) of the method according to the present invention (cf. experiments 11-19 in table 1) allows (vs. comparative test results without the use of phase transfer catalyst, cf. experiments 1-10 in table 1) performing the reaction at room temperature with a high conversion rate in a shortened reaction time, a low amount of undesired impurities being produced and a reduced portion of undesired epimer of the compound of formula V being produced, or the occurrence of undesired epimer of the compound of formula V may completely be avoided in preferred variations of the reaction of step S2).

The use of a phase transfer catalyst in a reaction of step S2) of the method according to the present invention therefore increases efficiency, yield and/or purity of the desired product of step S2), i.e. of the compound of formula V.

## Claims

1. Method of making one or more chemical compounds useful in pharmaceutical syntheses, the method comprising the following step:
S2) providing or preparing a compound of formula VI 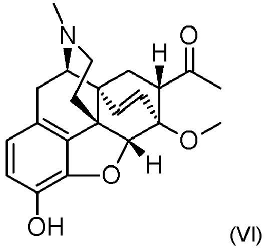
and reacting the compound of formula VI
in a liquid reaction medium comprising one or more non-halogenated organic solvents, in a total amount of ≥ 85 wt.-%, relative to the total weight of the liquid reaction medium,
wherein the one or more non-halogenated organic solvents are selected from the group consisting of N,N-dimethyl formamide, γ-butyrolactone, N-methyl-2-pyrrolidone, N-butyl-2-pyrrolidone and dihydrolevoglucosenon,
in the presence of a base and
in the presence of a phase transfer catalyst,
with an agent suitable for introducing to the free hydroxyl group of the compound of formula VI, a protective group PG which can be removed by hydrogenolysis,
to receive a compound of formula V 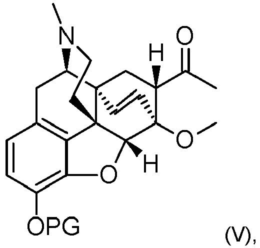
wherein PG is a protective group which can be removed by hydrogenolysis.

2. Method according to claim 1, wherein
- the liquid reaction medium used in step S2) comprises the one or more non-halogenated organic solvents, preferably comprises N,N-dimethyl formamide, in a total amount of ≥ 90 wt.-%, preferably of ≥ 95 wt.-%, relative to the total weight of the liquid reaction medium;
and/or
- the one or at least one of the more non-halogenated organic solvents is N,N-dimethyl formamide,
and/or
- the base has a pK_{b} at 25 °C of ≤ 4 and/or is selected from the group consisting of alkali metal carbonates, earth alkali carbonates and mixtures thereof;
and/or
- reacting in step S2) the compound of formula VI with the agent suitable for introducing, to the free hydroxyl group of the compound of formula VI, a protective group PG which can be removed by hydrogenolysis, is carried out at a temperature not exceeding 40 °C, more preferably at a temperature not exceeding 30 °C;
and/or
- the phase transfer catalyst comprises or is a quarternary ammonium salt, preferably selected from the group consisting of tetrabutyl ammonium chloride, tetrabutyl ammonium bromide, tetrabutyl ammonium iodide, benzyltriethylammonium chloride; benzyltriethylammonium bromide, benzyltriethylammonium iodide and mixtures thereof, more preferably the phase transfer catalyst comprises or is tetrabutyl ammonium bromide;
and/or
- the compound of formula VI is reacted with an agent suitable for introducing a protective group PG selected from the group consisting of benzyl, p-methoxybenzyl, 3,4-dimethoxybenzyl, (4-methoxyphenyl)diphenylmethyl and triphenylmethyl, preferably wherein the compound of formula VI is reacted with an agent suitable for introducing a benzyl protective group, preferably selected from the group consisting of benzyl chloride, benzyl bromide and mixtures thereof
and/or
- the protective group PG in the compound of formula V is a protective group which can be removed by hydrogenolysis, preferably PG is a protective group selected from the group consisting of benzyl, p-methoxybenzyl, 3,4-dimethoxybenzyl, (4-methoxyphenyl)diphenylmethyl and triphenylmethyl, more preferably PG is a benzyl protective group

3. Method according to any of the preceding claims, wherein the compound of formula VI is reacted with an agent suitable for introducing a protective group PG, preferably selected from the group consisting of benzyl chloride, benzyl bromide and mixtures thereof, and wherein any excess agent suitable for introducing the protective group PG is decomposed by heating to a temperature in the range of 50 to 90 °C, preferably of 60 to 80 °C, in the presence of the base, once the compound of formula V has formed.

4. Method according to any of the preceding claims, wherein the method further comprises the additional step:
S3) reacting a compound of formula V as defined in step S2) of claim 1, preferably as received in step S2), wherein PG is a protective group which can be removed by hydrogenolysis, preferably wherein PG is a protective group selected from the group consisting of benzyl, p-methoxybenzyl, 3,4-dimethoxybenzyl, (4-methoxyphenyl) diphenylmethyl and triphenylmethyl, more preferably wherein PG is a benzyl protective group,
in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium;
wherein preferably the one or more non-halogenated organic solvents are selected from the group consisting of N,N-dimethyl formamide, γ-butyrolactone, N-methyl-2-pyrrolidone, N-butyl-2-pyrrolidone and dihydrolevoglucosenon, more preferably wherein the one or at least one of the more non-halogenated organic solvents is N,N-dimethyl formamide;
with a de-methylating agent, to receive a compound of formula IV 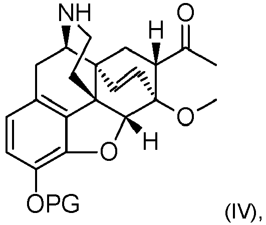
wherein PG has the above meaning.

5. Method according to any of the preceding claims, preferably according to claim 3 or 4, wherein the liquid mixture comprising the compound of formula V as received after step S2) is directly used for the reaction in step S3), preferably without prior isolation or purification of the compound of formula V obtained in step S2).

6. Method according to any of the preceding claims, wherein the method further comprises the additional step:
S4) reacting a compound of formula IV as defined in step S3) of claim 4, preferably as received in step S3),
in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium,
wherein preferably the one or more non-halogenated organic solvents are selected from the group consisting of N,N-dimethyl formamide, γ-butyrolactone, N-methyl-2-pyrrolidone, N-butyl-2-pyrrolidone and dihydrolevoglucosenon, more preferably wherein the one or at least one of the more non-halogenated organic solvents is N,N-dimethyl formamide;
with a halogeno-methylcyclopropane, to receive a compound of formula III 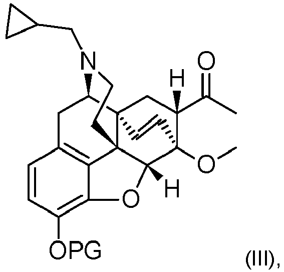
wherein PG is a protective group which can be removed by hydrogenolysis, preferably wherein PG is a protective group selected from the group consisting of benzyl, p-methoxybenzyl, 3,4-dimethoxybenzyl, (4-methoxyphenyl)diphenylmethyl and triphenylmethyl, more preferably wherein PG is a benzyl protective group.

7. Method according to any of the preceding claims, wherein the method further comprises the additional step:
S1) providing or preparing oripavine of formula VII 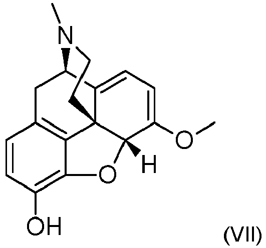
and reacting the compound of formula VII
in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium;
wherein preferably the one or at least one of the more non-halogenated organic solvents is ethanol;
with 3-buten-2-one under conditions of a Diels-Alder reaction, preferably under conditions of temperature and pressure so that boiling of the liquid reaction medium is avoided,
to receive a compound of formula VI as defined in step S2) of claim 1.

8. Method according to any of the preceding claims, wherein the method further comprises the additional step:
S5) reacting a compound of formula III as defined in step S4) of claim 6, preferably as received in step S4),
in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium;
wherein preferably the one or more non-halogenated organic solvents are selected from the group of dialkylethers,
and/or are selected from the group consisting of methyl tert.-butyl ether; diethyl ether; 1,4-dioxan and tetrahydrofuran, preferably are selected from the group consisting of methyl tert.-butyl ether; diethyl ether and 1 ,4-dioxan, more preferably wherein the one or at least one of the more non-halogenated organic solvents is methyl tert.-butyl ether;
with a tert.-butyl magnesium halide, preferably tert.-butyl magnesium chloride, under conditions of a Grignard reaction, to receive a compound of formula II

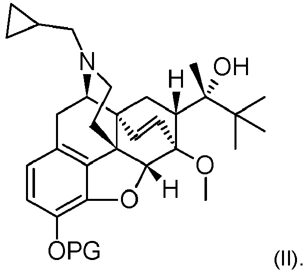

9. Method according to any of the preceding claims, wherein the method further comprises the additional step:
S6) reacting a compound of formula II as defined in step S5) of claim 8, preferably as received in step S5),
in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium;
wherein preferably the one or at least one of the more non-halogenated organic solvents is ethanol;
under conditions suitable for removing the protective group PG by hydrogenolysis,,
to receive buprenorphine of formula I

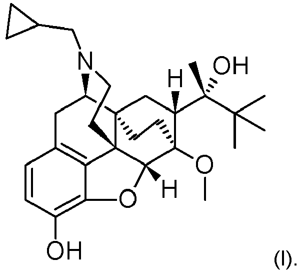

10. Method according to any of the preceding claims, wherein the reaction in each of the steps S1), S2), S3), S4), S5) and S6) is carried out in a liquid reaction medium comprising one or more non-halogenated organic solvents, in a total amount of ≥ 85 wt.- %, preferably of ≥ 90 wt.-%, more preferably of ≥ 95 wt.-%, relative to the total weight of the liquid reaction medium.

11. Method according to any of the preceding claims, wherein the reaction in each of the steps S2) and S3), or in each of the steps S2), S3) and S4), is carried out in a liquid reaction medium comprising
one or more non-halogenated organic solvents in a total amount of ≥ 85 wt.-%, preferably of ≥ 90 wt.-%, and still more preferably of ≥ 95 wt.-%, relative to the total weight of the liquid reaction medium,
and
the one or more non-halogenated organic solvents are selected in each case from the group consisting of N,N-dimethyl formamide, γ-butyrolactone, N-methyl-2-pyrrolidone, N-butyl-2-pyrrolidone and dihydrolevoglucosenon,
preferably wherein the reaction in each of the steps S2) and S3), or in each of the steps S2), S3) and S4), is carried out in a liquid reaction medium wherein at least one of the one or more non-halogenated organic solvents present in the liquid reaction medium is the same in each of the steps S2) and S3), or in each of the steps S2), S3) and S4), and is present in a total amount of ≥ 85 wt.-%, preferably of ≥ 90 wt.-%, more preferably of ≥ 95 wt.-%, relative to the total weight of the totality of non-halogenated organic solvents of the liquid reaction medium in the respective step S2), S3) and/or S4).

12. Method according to any of the preceding claims, wherein the de-methylating agent used in step S3) comprises or is an azodicarboxylic acid alkyl ester of formula IX
R¹-OOC-N=N-COOR² (IX),
wherein R¹ and R² are independently selected from the group consisting of linear alkyl with 1 to 6 carbon atoms and branched alkyl with 1 to 6 carbon atoms, preferably selected from the group consisting of methyl, ethyl, n-propyl, isopropyl, n-butyl, sec.-butyl, tert.-butyl, pentyl and hexyl, wherein more preferably the azodicarboxylic acid alkyl ester of formula IX comprises or is diisopropyl azodicarboxylate,
preferably wherein the de-methylating agent used in step S3) comprises or is diisopropyl azodicarboxylate and the one or at least one of the more non-halogenated organic solvents used in the liquid reaction medium in step S3) comprises or is dimethyl formamide,
more preferably wherein the de-methylating agent used in step S3) comprises or is diisopropyl azodicarboxylate and the reaction in step S3) is carried out in a liquid reaction medium comprising ≥ 90 wt.-%, preferably ≥ 95 wt.-%, of N,N-dimethyl formamide, relative to the total weight of the liquid reaction medium.

13. Method according to any of the preceding claims, preferably according to claim 7 and/or to claim 9, wherein the reaction in each of the steps S1) and S6) is carried out in a liquid reaction medium comprising ethanol in a total amount of ≥ 85 wt.-%, preferably of ≥ 90 wt.-%, more preferably of ≥ 95 wt.-%, relative to the total weight of the liquid reaction medium.

14. Method according to any of the preceding claims, wherein
- the protective group PG in each of compounds of formula II, III, IV and V is the same and is selected from the group consisting of benzyl, p-methoxybenzyl, 3,4-dimethoxybenzyl, (4-methoxyphenyl)diphenylmethyl and triphenylmethyl, preferably wherein the protective group PG in each of compounds of formula II, III, IV and V is benzyl;
and/or
- the method comprises at least steps S2), S3) and preferably S4);
and/or
- the method is a method of making buprenorphine from oripavine, or the method is part of a method of making buprenorphine from oripavine.

15. Method of making buprenorphine from oripavine according to any of the preceding claims, comprising the following steps:
S1) providing or preparing oripavine of formula VII 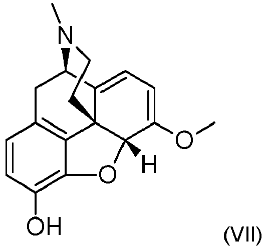
and reacting the compound of formula VII
with 3-buten-2-one under conditions of a Diels-Alder reaction, preferably under conditions of temperature and pressure so that boiling of the liquid reaction medium is avoided,
to receive a compound of formula VI 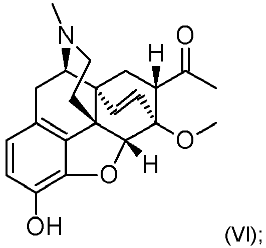
S2) reacting the compound of formula VI as received in step S1)
at a temperature not exceeding 30 °C, in the presence of a base, preferably wherein the base has a pK_{b} at 25 °C of ≤ 4 and more preferably is selected from the group consisting of alkali metal carbonates, earth alkali carbonates and mixtures thereof; and
in the presence of a phase transfer catalyst,
with an agent suitable for introducing a protective group PG which can be removed by hydrogenolysis, preferably with an agent suitable for introducing a benzyl protective group PG,
to receive a compound of formula V 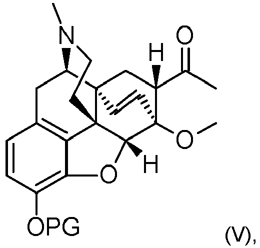
wherein PG is a protective group which can be removed by hydrogenolysis, preferably benzyl;
S3) reacting the compound of formula V as received in step S2) with a de-methylating agent, preferably with an azodicarboxylic acid alkyl ester of formula IX
R¹-OOC-N=N-COOR² (IX),
wherein R¹ and R² are independently selected from linear or branched alkyl with 1 to 6 carbon atoms, preferably with diisopropyl azodicarboxylate,
to receive a compound of formula IV 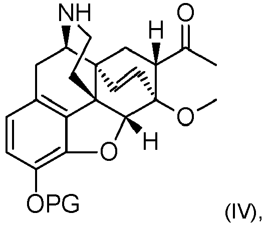
wherein PG has the above meaning;
S4) reacting the compound of formula IV as received in step S3) with a halogeno-methylcyclopropane, preferably with bromomethyl cyclopropane, to receive a compound of formula III 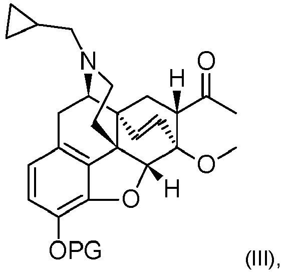 wherein PG has the above meaning;
S5) reacting the compound of formula III as received in step S4) with a tert.-butyl magnesium halide, preferably with tert.-butyl magnesium chloride, under conditions of a Grignard reaction, to receive a compound of formula II 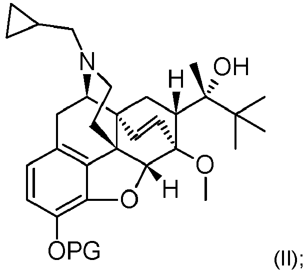 and
S6) reacting the compound of formula II as received in step S5) under conditions suitable to remove the protective group PG by hydrogenolysis,
to receive buprenorphine of formula I 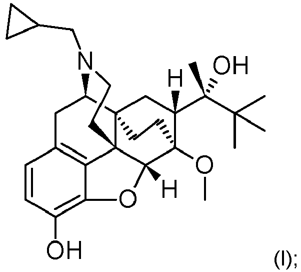 wherein
- each of the steps S1), S2), S3), S4), S5) and S6) is carried out in a liquid reaction medium comprising one or more non-halogenated organic solvents, in a total amount of ≥ 85 wt.-%, preferably of ≥ 90 wt.-%, more preferably of ≥ 95 wt.-%, relative to the total weight of the liquid reaction medium,
and/or
- the reaction in each of the steps S2), S3) and S4) is carried out in a liquid reaction medium comprising one or more non-halogenated organic solvents, selected in each case from the group consisting of N,N-dimethyl formamide, γ-butyrolactone, N-methyl-2-pyrrolidone, N-butyl-2-pyrrolidone and dihydrolevoglucosenon, preferably wherein the reaction in each of the steps S2), S3) and S4) is carried out in a liquid reaction medium comprising the same one or more non-halogenated organic solvents, more preferably wherein the reaction in each of the steps S2), S3) and S4) is carried out in a liquid reaction medium comprising N,N-dimethyl formamide in a total amount of ≥ 85 wt.-%, preferably of ≥ 90 wt.-%, more preferably of ≥ 95 wt.-%, relative to the total weight of the liquid reaction medium;
and/or
- wherein the reaction in each of the steps S1) and S6) is carried out in a liquid reaction medium comprising ethanol in a total amount of ≥ 85 wt.-%, preferably of ≥ 90 wt.- %, more preferably of ≥ 95 wt.-%, relative to the total weight of the liquid reaction medium.
